Europäisches Patentamt

European Patent Office

Office européen des brevets

(19)

(11) Veröffentlichungsnummer: **0 208 743 B1**

(12) **EUROPÄISCHE PATENTSCHRIFT**

(45) Veröffentlichungstag der Patentschrift: **14.10.92**

(51) Int. Cl.5: **A61K 39/104**, C07K 3/12, C07K 15/04

(21) Anmeldenummer: **86900707.0**

(22) Anmeldetag: **13.01.86**

(86) Internationale Anmeldenummer: **PCT/AT86/00002**

(87) Internationale Veröffentlichungsnummer: **WO 86/03974 (17.07.86 86/17)**

Die Akte enthält technische Angaben, die nach dem Eingang der Anmeldung eingereicht wurden und die nicht in dieser Patentschrift enthalten sind.

(54) POLYDISPERSE NATIVE PSEUDOMONAS-FLAGELLA(H)-ANTIGENE (FAg) UND VERFAHREN ZU IHRER GEWINNUNG.

(30) Priorität: **14.01.85 AT 72/85**

(43) Veröffentlichungstag der Anmeldung: **21.01.87 Patentblatt 87/04**

(45) Bekanntmachung des Hinweises auf die Patenterteilung: **14.10.92 Patentblatt 92/42**

(84) Benannte Vertragsstaaten: **AT BE CH DE FR GB IT LI LU NL SE**

(56) Entgegenhaltungen: **EP-A- 0 048 422**

**Infection and Immunity, Band 35, Nr. 1, Januar 1982,T.C. Montie et al.: "Flagellar Preparations from Pseudomonas aeruginosa: Isolation and Characterization", Seiten 281-288**

(73) Patentinhaber: **IMMUNO Aktiengesellschaft Industriestrasse 67 A-1221 Wien(AT)**

(72) Erfinder: **MONTIE, Thomas, C./University of Tennessee Dep.of Microbiology Walters Life Science Building Knoxville,TN 37996-0854(US)**
Erfinder: **DORNER, Friedrich Peterlinigasse 17 A-1230 Wien(AT)**
Erfinder: **McDONEL, James, I. 814 Cottage Grove Avenue South Bend, IN 46616(US)**
Erfinder: **MITTERER, Artur Am Markt 30 A-2304 Orth/Donau(AT)**

Chemical Abstracts, Band 103, Nr. 17, 28. Oktober 1985, Columbus, Ohio(US),J.S. Allison et al.: "Electrophoretic separation and molecular weight characterization of Pseudomonas aeruginosa H-antigen flagellins", siehe Seite 550, Zusammenfassung 139921q

Biological Abstracts, Band 78, 1984,R.A. Ansorg et al.: "Differentiation of the major flagellar antigens of Pseudomonas aeruginosa by the slide coagglutination technique", siehe Zusammenfassung 71429

Chemical Abstracts, Band 99, Nr. 23, 5. Dezember 1983, Columbus, Ohio (US),T.C: Montie et al.: "Isolation and characterization of flagellar prepararions from Pseudomonas species", siehe Seite 429, Zusammenfassung 191185r

Abstracts of the Annual Meeting of the American Society of Microbiology, Band 81, 1-6 März 1981,T.C. Montie et al.: "Protection of burned pseudomonas infected mice using flagellar preparations", siehe Seite 17, Zusammenfassung B 17

The Proteins (Third Edition) Vol. V (eds. Neurath & Hill) Acad. Press, 1982, Seiten 15-18

Med. Microbiol. Immunol. 168 (1980), 217-226

Zbl. Bakt. Hyg., I Abt. Orig. A 246 (1980) 363-372

J. Med. Microbiol. 14 (1981) 251-260

Anal. Biochem. 119 (1982) 115-119

(74) Vertreter: **Wolfram, Gustav, Dipl.-Ing.** **Patentanwälte Sonn, Pawloy, Weinzinger & Wolfram, Riemergasse 14** **A-1010 Wien(AT)**

**Beschreibung**

Die Erfindung betrifft polydisperse native Pseudomonas-Flagella(H)-Antigene (FAg) und ein Verfahren zu ihrer Gewinnung aus Pseudomonas aeruginosa-Bakterienkulturen.

Bakterium Pseudomonas aeruginosa ist ein opportunistischer, pathogener Keim, der häufig bei Krankenhausinfektionen auftritt, hauptsächlich bei Patienten mit geschwächter Immunabwehr, wie bei Verbrennungspatienten, bei Personen, die an cystischer Fibrose leiden oder organische Fehlfunktionen aufweisen, und bei Tumorpatienten. Antibiotika sind gegen Pseudomonasinfektionen infolge des Auftretens von Resistenzen nur beschränkt wirksam, weshalb man bemüht ist, immunologische Methoden zur Bekämpfung von Infektionen durch Pseudomonas aeruginosa anzuwenden.

Infektionen können durch eine Vielzahl von Stämmen ausgelöst werden, die O-Gruppenantigene und H-Antigene produzieren. Gemäß dem H-Antigenschema nach Ansorg (Zbl. Bakt. Hyg. I. Abt. Orig. A 242, 228 - 238 (1978) wird unter Verwendung der indirekten Immunfluoreszenztechnik bei Pseudomonas aeruginosa ein komplexes Flagella(H)-Antigen a mit den Partialantigenen $a_0$, $a_1$, $a_2$, $a_3$, $a_4$ und ein uniformes Flagella-(H)-Antigen b differenziert. Die Partialfaktoren $a_0$ - $a_4$ sind unabhängige Determinanten, so daß ein flagellares Antigenschema mit mehreren H-Typen resultiert. O-Gruppen und H-Typ zeigen freie Kombinationen.

Zur Bereitung von Pseudomonas aeruginosa-Bakterienkulturen verwendbare Stamme und die produzierten Antigene werden in der folgenden Tabelle angeführt.

| | Stamm | | H-Typ |
|---|---|---|---|
| 1 | 170001 | – | b |
| | M-2 | – | b |
| 2 | 5142 | – | $a_0$ |
| 3 | 5940 | – | $a_0$, $a_2$ |
| 4 | 5939 | – | $a_0$, $a_3$ |
| 5 | 5933 | – | $a_0$, $a_1$, $a_2$ |
| | 1210 | – | $a_0$, $a_1$, $a_2$ |
| | 16990 | – | $a_0$, $a_1$, $a_2$ |
| 6 | 170018 | – | $a_0$, $a_3$, $a_4$ |

Isolierte Filamente der Flagellen-Antigene, die durch Schütteln, Homogenisation und anschließende Zentrifugation gewonnen werden können (R. Ansorg, W. Schmitt, Med. Microbiol. Immunol. (1980) 168: 217-226), bestehen aus Flagellen und Flagellenbruchstücken, vereint in einem Komplex bestehend aus Lipopolysacchariden (LPS) und Verunreinigungen aus dem Nährmedium; solche Präparationen sind naturgemäß pyrogen und ungeeignet für die Anwendung am Menschen.

Es ist bekannt, zur Bekämpfung von Pseudomonasinfektionen Pseudomonasvakzime herzustellen, wobei als Ausgangsmaterialien Pseudomonas aeruginosa-Bakterienmassen und/oder Kulturfiltrate verwendet werden, die unter Züchtung der Mikroorganismen auf Oberflächenkulturen oder submers in komplexen Nährmedien gewonnen wurden. Bei diesen komplexen Nährmedien wurden neben einer Kohlenstoff- und Energiequelle (meist Kohlenhydrate) und essentiellen Nährsalzen verschiedenste Extrakte und/oder Hydrolysate tierischer, mikrobieller oder pflanzlicher Proteine (sogenannte Peptone) verwendet. Derartige Nährlösungssupplemente sind in ihrer genauen Zusammensetzung nicht definiert und zudem von Charge zu Charge variabel. Sie enthalten neben Aminosäuren auch unvollständig abgebaute Proteinbruchstücke und undefinierte Komplexe derselben und dienen im wesentlichen zur Deckung des Aminosäure- und Wuchsstoffbedarfes. Kulturüberstände sind deshalb immer reich an Substanzen nicht-bakteriellen Ursprungs, was den Nachteil hat, daß für die Bereitung eines Flagella(H)-Antigens von Pseudomonas aeruginosa immer mehrere der Kultivierungsstufe nachzustellende Trennstufen notwendig sind, um das Flagella(H)-Antigen soweit wie möglich von aus dem Nährmedium stammenden Verunreinigungen zu befreien.

Die Abtrennung des rohen flagellaren Materials aus der Bakteriensuspension wurde durch sogenanntes "Shearing", d.i. Einwirken von Scherkräften auf die Bakteriensuspension, in einem Mixer vorgenommen. Hierauf wird bei 15.000 x g - 18.000 x g abzentrifugiert, das Pellet verworfen und der Überstand, der die rohe Flagellenpräparation enthält, einer Zentrifugalbeschleunigung von mindestens 40.000 x g, 1 Stunde,oder 100.000 x g, 20 min, unterworfen. Dabei fällt das rohe Antigen in Form von Pellets an. Es enthält, wie schon vorher erwähnt, u.a. Lipopolysaccharide (LPS), Nucleinsäuren, verschiedene Salze, Polysaccharide und nicht flagellare Proteine, die die Effizienz und Verträglichkeit daraus hergestellter Vakzine ungünstig beeinflussen. Flageila(H)-Antigen konnte bislang noch nicht rein isoliert werden (T.L. Pitt, J. Med. Microbiol. (1981), 14: 251 - 260).

Die Erfindung bezweckt die Vermeidung der geschilderten Nachteile und Schwierigkeiten und stellt sich die Aufgabe, polydisperse native Flagella(H)-Antigene (FAg) in hoher Reinheit, frei von pyrogenen Substanzen, bereitzustellen.

Diese hochreinen FAg-Antigene gemäß der Erfindung liegen in polymerer Form vor mit

(i) einem Diffusionskoeffizienten von $D = 8,5 \times 10^{-9}$ (cm$^2$/s),

(ii) einem hydrodynamischen Radius von $R = 2,5 \times 10^{-5}$ cm,

(iii) einer Dichte von $d = 1,28$ g/cm$^3$ und

(iv) einer weitgehenden Freiheit von LPS, das ist ein Anteil von weniger als 1 %,

und bestehen aus monomeren Bestandteilen, wobei jeder monomere Bestandteil

a) folgende Aminosäuren: Asparaginsäure (Asp), Threonin (Thr), Serin (Ser), Glutaminsäure (Glu), Glycin (Gly), Alanin (Ala), Valin (Val), Isoleucin (Ile), Leucin (Leu), Tyrosin (Tyr), Phenylalanin (Phe), Lysin (Lys), Arginin (Arg) und gegebenenfalls Tryptophan (Trp) enthält,

b) die N-terminale Aminosäuresequenz Alanin (Ala) - Leucin (Leu) - Threonin (Thr) - Valin (Val) - Asparagin (Asn) - Threonin (Thr) - Asparagin (Asn) - Isoleucin (Ile) - Alanin (Ala) - aufweist, und

c) ein Molekulargewicht zwischen 43.500 und 53.050 aufweisen und

d) frei sind von Prolin, Methionin, Halb-Cystin und Histidin.

Im einzelnen werden erfindungsgemäß sechs spezifische H-Serotypen charakterisiert, u.zw.

- das Flagella(H)-Antigen vom H-Serotyp $a_0$, $a_3$, $a_4$, dessen monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 64 : 33 : 35 : 42 : 44 : 68 : 29 : 29 : 37 : 3 : 10 : 19 : 15 enthält und ein Molekulargewicht von 43.500 aufweist;

- das Flagella(H)-Antigen vom H-Serotyp $a_0$, $a_3$, dessen monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 69 : 35 : 38 : 44 : 47 : 73 : 30 : 30 : 60 : 3 : 12 : 21 : 16 enthält und ein Molekulargewicht von 46.700 aufweist;

- das Flagella(H)-Antigen vom H-Serotyp $a_0$, dessen monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 50 : 49 : 49 : 49 : 89 : 37 : 29 : 44 : 5 : 14 : 17 : 16 enthält und ein Molekulargewicht von 52.720 aufweist;

- das Flagella(H)-Antigen vom H-Serotyp b, dessen monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 48 : 48 : 49 : 51 : 91 : 38 : 30 : 43 : 4 : 13 : 18 : 18 enthält und ein Molekulargewicht von 53.050 aufweist;

- das Flagella(H)-Antigen vom H-Serotyp $a_0$, $a_1$, $a_2$, dessen monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 76 : 44 : 40 : 52 : 50 : 81 : 32 : 32 : 41 : 4 : 12 : 20 : 18 enthält und ein Molekulargewicht von 51.250 aufweist;

- das Flagella(H)-Antigen vom H-Serotyp $a_0$, $a_2$, dessen monomore Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 68 : 41 : 37 : 46 : 44 : 73 : 29 : 29 : 37 : 3 : 10 : 16 : 16 enthält und ein Molekulargewicht von 45.900 aufweist.

Die Erfindung umfaßt weiters ein Verfahren zur Gewinnung der bezeichneten polydispersen nativen Pseudmonas Flagella(H)-Antigene aus Pseudmonas aeruginosa Bakterienkulturen, welches darin besteht, daß die Bakterienkultur desintegriert, vorzugsweise einem "Shearing"-Prozeß unterworfen und fraktioniert wird, nach Zusatz eines Detergens einer chromatographischen Behandlung bzw. Reinigung unterworfen wird, wobei für die chromatographische Behandlung insbesondere ein Molekularsieb eingesetzt wird, welches mit dem Detergens äquilibriert ist, und daß das chromatographisch behandelte und gereinigte flagellare Antigen einer weiteren chromatographischen Reinigung zur Entfernung von vorhandenem Detergens unterworfen wird, wobei Flagella-Antigene mit einer Reinheit von mehr als 98 % und einem Anteil an pyrogenen Substanzen (LPS) von weniger als 1 % erhalten werden.

4

Durch den Zusatz des Detergens wird die Trennbarkeit des Antigens von der Bakterienmasse bewirkt und die sich dann in Lösung befindlichen Antigene abgetrennt. Als Detergens ist vorteilhaft ein Salz der Gallensäure, insbesondere Deoxycholat, geeignet.

Durch die chromatographische Behandlung bzw. Reinigung werden die als Verunreinigungen vorhandenen Makromoleküle, insbesondere die Lipopolysaccharide, Nucleinsäuren, Salze, Polysaccharide u.a. zurückgehalten.

Eine zu empfehlende Modifikation des erfindungsgemäßen Verfahrens besteht darin, daß die desintegrierte Bakterienkultur vor der chromatographischen Behandlung einer Vorreinigung unterworfen wird, wobei Bakterien und mikroskopisch sichtbare Bakterienteilchen aus der Kultur entfernt werden. Diese Vorreinigung kann durch Zentrifugieren mit einer Zentrifugalbeschleunigung bis zu 5.000 x g vorgenommen werden; das abgeschiedene Sediment wird verworfen und der Überstand weiter verarbeitet.

Eine weitere vorteilhafte Modifikation des erfindungsgemäßen Verfahrens besteht darin, daß auch die zweite chromatographische Reinigung mit einem Molekularsieb, wie Sephadex (Warenzeichen) oder mit einem nicht polaren Polystyroladsorptionsgel, wie BIO-BEADS SM-4 (Warenzeichen), durchgeführt wird.

Das erfindungsgemäße Verfahren wird durch das folgende Ausführungsbeispiel näher erläutert:

Eine Bakterienkultur von Pseudomonas aeruginosa M-2 (ausgewählt aus den einleitend angeführten Bakterienstämmen) wurde in einer Nährlösung folgender Zusammensetzung:

| | |
|---|---|
| Dinatriumsuccinat | 4,05 g/l, |
| Dikaliummonohydrogenphosphat | 7 g/l, |
| Kaliumdihydrogenphosphat | 3 g/l, |
| Ammoniumhydrogenphosphat | 1 g/l, |
| Magnesiumsulfat . 7 $H_2O$ | 0,05 g/l, |
| Eisen(III)chlorid | 0,0025 g/l, |

entwickelt, bis die Zelldichte 2 - 3 x $10^9$ Zellen/ml betrug, und bei einer Verdünnungsrate von 0,2 $\mu$ während einer Fermentationsdauer von 96 Stunden bei einem Sauerstoffpartialdruck von 10 % ($pO_2$) und unter Rühren konstant gehalten.

Die aus dem Fermenter kontinuierlich abgezogene Bakterienkultur wurde in einer mit dem Fermenter zusammenarbeitenden Ultrazentrifuge mit einer Rotoraufnahmekapazität von 660 ml (0,45 kg Feuchtmasse) bei einer Zentrifugalbeschleunigung von 18,000 x g aufgearbeitet, wobei bei Verwendung einer verdünnungsrate von 0,2 $\mu$ nach 96 Stunden soviel Biomasse angesammelt wurde, daß daraus nach dem Einwirken von Scherkräften im Mixer, Abtrennung von Zellen und Zellbruchstücken bei 15.000 x g - 18.000 x g aus dem Pellet der Ultrazentrifuge 149 mg rohes flagellares Antigen erhalten wurde.

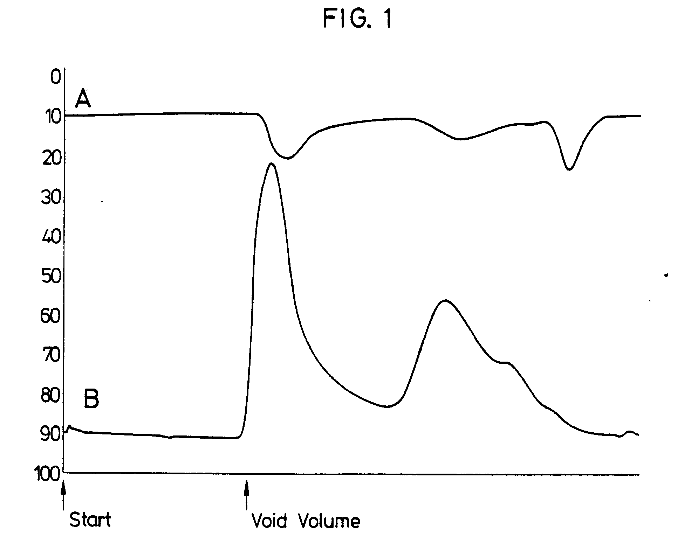

18 mg des erhaltenen rohen flagellaren Antigens wurden in 12 ml einer 30 mM Tris-HCl-Pufferlösung mit einem pH-Wert von 7,0, der 20 mg/ml Deoxycholat zugesetzt waren, gelöst und auf eine 16 x 16 cm Säule aufgetragen, die mit Sephacryl S-1000 gefüllt und mit dem 30 mM Tris-HCl-Puffer, pH 7,0, dem 2 mg/ml Deoxycholat zugesetzt waren, äquilibriert war. Hierauf wurden 2 ml Fraktionen gesammelt und das Elutionsdiagramm durch Verfolgung der Extinktion bei 280 und 254 nm aufgenommen. Der Kolonnenlauf ist im beiliegenden Diagramm (Fig. 1) veranschaulicht. Die mit A bezeichnete Kurve ist bei einer Extinktion bei 254 nm und bei einer Küvette von 1 mm Durchmesser aufgenommen; die mit B bezeichnete Kurve ist bei einer Extinktion bei 280 nm und bei einer Küvette von 20 mm Durchmesser aufgenommen. In der Absorptionskurve bei 280 nm ist der Antigen-Peak deutlich zu ersehen.

Die das gereinigte Flagella(H)-Antigen enthaltende Lösung wurde sodann einem zweiten Reinigungsschritt unterworfen, um das von der ersten Reinigungsstufe verbliebene Deoxycholat zu entfernen, zu welchem Zweck entweder ein Molekularsieb mit kleinerem Vernetzungsgrad, z.B. Sephadex G-25, verwendet wird oder ein Adsorptionsgel wie BIO-BEADS SM-4. Bei dieser Behandlung wurde das DOC abgetrennt und das flagellare Antigen in einer Reinheit von mehr als 98 % und einem Anteil an pyrogenen Substanzen (LPS) von weniger als 1 % erhalten.

In gleicher Weise können Bakterienkulturen der übrigen einleitend angeführten Stämme entwickelt und daraus in analoger Weise die einzelnen H-Typ-Antigene in reiner Form erhalten werden. Dabei hat sich gezeigt, daß die Ausbeute an FAg von der Wahl der Stämme abhängig ist. Gewisse Stämme, wie 5933 ($a_0$, $a_1$, $a_2$) und 1210 ($a_0$, $a_1$, $a_2$) ergeben größere Zellausbeuten als 170001 (b) und M-2 (b).

Die Kennwerte der einzelnen erfindungsgemäß charakterisierten Flagella(H)-Antigene, welche polymere Verbindungen sind und aus monomeren Bestandteilen bestehen, wurden in folgender Weise bestimmt:

A) Aminosäureanalyse

Für die Analyse der gesamten Aminosäurezusammensetzung der Flagella(H)-Antigene wurden Proben hydrolysiert, um die Peptidbindungen aufzubrechen. Hierbei wurde die Standardmethode zur Proteinhydrolyse (6 N HCl, 110°C, 22 Stunden) angewendet (C.H.W. Hirs, Methods in Enzymology, S.P. Colowick, N.O. Kaplan, Editors-in-chief, Volume XI, Enzyme Structure, p 59-62, C.H.W. Hirs; ed. 1967, Academic Press).

Die Untersuchungen wurden in einem Beckman System 6300 Aminosäureanalysator durchgeführt. Aufgrund der Ergebnisse der Aminosäurenzusammensetzung wurde das Molekulargewicht der monomeren Untereinheiten berechnet.

B) Reinheit und Molekulargewicht

Die Reinheit bzw. Freiheit von Verunreinigungen der erfindungsgemäßen Antigene wurde mittels Polyacrylamidgelelektrophorese in Natriumdodecylsulfat (A.L. Shapiro, E. Vinuela, J. Maizel, Biochem. Biophys. Res. Commun. (1967), 28: 815) überprüft.

Das Elektropherogramm wurde mit der "Silver-Staining"-Methode gefärbt (Merril, C.R., Goldman, D., Sedman, S.A. and Ebert, M.H., Science (1981), 211: 1437). Es zeigte sich bei allen untersuchten Flagella-Antigenen eine einzige Bande, die entsprechend der nachstehend angeführten Methode aufgrund ihrer Molekulargewichte eine bestimmte Wanderungsstrecke zurückgelegt hatte. Es waren keine Hinweise auf LPS feststellbar.

Polyacrylamidgelelektrophorese in Natriumdodecylsulfat nach der Modifikation nach Osborn und Weber (Weber, K. und Osborn, M. (1969) J. Biol. Chem. 244: 4406) wurde dazu verwendet, die gereinigten Flagella-Antigene in ihre monomeren Untereinheiten aufzuspalten und deren Molekulargewichte zu bestimmen.

In der folgenden Tabelle sind die den einzelnen Stämmen zugeordneten Flagella(H)-Antigene vom jeweiligen H-Serotyp mit der entsprechenden Zusammensetzung ihrer Aminosäuren und ihrem Molekulargewicht aufgelistet. Gegebenenfalls vorhandenes Tryptophan ist in der Tabelle nicht angegeben, weil es während der Hydrolyse ganz oder teilweise zerstört wird.

**Anzahl AS / Molekül Flagellin**

Stämme mit zugehörigem H-Serotyp

| Aminosäuren | 170018 a0,a3,a4 | 5939 a0, a3 | 5142 a0 | M-2 b | 1210 a0,a1,a2 | 5940 a0,a2 |
|---|---|---|---|---|---|---|
| Asparaginsäure | 64 | 69 | 74 | 74 | 76 | 68 |
| Threonin | 33 | 35 | 50 | 48 | 44 | 41 |
| Serin | 35 | 38 | 49 | 48 | 40 | 37 |
| Glutaminsäure | 42 | 44 | 49 | 49 | 52 | 46 |
| Glycin | 44 | 47 | 49 | 51 | 50 | 44 |
| Alanin | 68 | 73 | 89 | 91 | 81 | 73 |
| Valin | 29 | 30 | 37 | 38 | 32 | 29 |
| Isoleucin | 29 | 30 | 29 | 30 | 32 | 29 |
| Leucin | 37 | 60 | 44 | 43 | 41 | 37 |
| Tyrosin | 3 | 3 | 5 | 4 | 4 | 3 |
| Phenylalanin | 10 | 12 | 14 | 13 | 12 | 10 |
| Lysin | 19 | 21 | 17 | 18 | 20 | 16 |
| Arginin | 15 | 16 | 16 | 18 | 18 | 16 |
| Prolin | 0 | 0 | 0 | 0 | 0 | 0 |
| Methionin | 0 | 0 | 0 | 0 | 0 | 0 |
| Halb-Cystin | 0 | 0 | 0 | 0 | 0 | 0 |
| Histidin | 0 | 0 | 0 | 0 | 0 | 0 |
| $\Sigma$ = | 450 | 478 | 522 | 525 | 502 | 449 |
| MG = | 43.500 | 46.700 | 52.720 | 53.050 | 51.250 | 45.900 |

C) N-terminale Aminosäuresequenz

Mit einem Beckman-System 890 Protein/Peptide Sequencer wurde mit der "solid phase"-Methode (P. Edman, G. Begg, 1967, A protein sequenator, Eur.J.Biochem. 1:80-91) die Sequenz der ersten neun Aminosäuren vom N-terminalen Ende der gereinigten Flagella(H)-Antigene bestimmt. Bei allen Typen war die gleiche Sequenz festzustellen.

```
M-2
5142
5939        Ala-Leu-Thr-Val-Asn-Thr-Asn-Ile-Ala-
1210
170018
5940
```

D) Teilchendichte von Flagella(H)-Antigenen

Die Teilchendichte der erfindungsgemäßen Flagella(H)-Antigene wurde jeweils dadurch bestimmt, daß 1 ml einer Probenflüssigkeit (Flagella(H)-Antigen in aqua dest.) einem CsCl-Gradienten von d = 1,15 - 1,45 g/cm$^3$ überschichtet wurde.

Nach Zentrifugation bei 50.000 rpm in einem Beckman 70 Ti-Rotor für 16 Stunden wurden Fraktionen vom Boden der Zentrifugenröhrchen abgenommen und mittels Polyacrylamidgelelektrophorese in Natriumdodecylsulfat analysiert.

Alle untersuchten Flagella(H)-Antigen-Präparationen zeigen eine Dichte von d = 1,28 g/cm$^3$.

E) Quasielastische Lichtstreuung

Die Bestimmung beruht darauf, daß bei Einwirkung von Laserlicht auf eine makromolekulare Lösung das Licht von den Molekülen gestreut wird. Die Moleküle behalten im Lösungsmittel nie ihren momentanen Standpunkt ein, sondern bewegen sich aufgrund der Brown'schen Molekularbewegung. Bewegen sich die Moleküle z.B. vom Laser weg, so wird das gestreute Licht (gemäß dem Dopplereffekt) in seiner Frequenz etwas erniedrigt sein. Aus diesen Frequenzshifts können Informationen über die Diffusion der Moleküle abgeleitet und daraus der "hydrodynamische Radius" errechnet werden (Photon correlation spectroscopy and velocimetry, H.Z. Cummins and E.R. Pike, Plenum Press, N.Y., London, 1977).

Im Falle der Flagella(H)-Antigene repräsentiert der hydrodynamische Radius nicht den Radius des Moleküls, sondern einen mittleren Größenparameter. Dieser zeigt jedoch deutlich eine Abhängigkeit vom Alter der Probe.

Als Untersuchungsbedingungen wurden angewandt: Streuwinkel: 60°, Wellenlänge: 632,8 nm, Temperatur: 20°C. Als Resultat wurde für alle Flagella-Antigene: M-2, 5142, 5940, 5939, 1210, 170018 ein Diffusionskoeffizient von D = 8,5 x 10$^{-9}$ (cm$^2$/sec) und ein hydrodynamischer Radius von R = 2,5 x 10$^{-5}$ cm erhalten.

F) Immunologische Analysen

Herstellung von Immunseren:

Rohe, sowie hochgereinigte Flagella-Antigen-Präparationen wurden zur Immunisierung von weißen New Zealand Kaninchen verwendet. Das Immunisierungsschema entsprach der von Lagenaur und Agabian (J. Bacteriol. 128: 435-444, 1976) beschriebenen Methode. Dabei wird 1 ml bestehend aus einer 1:1-Mischung Flagellen-Antigen-Präparation (500 µg/ml) und komplettem Freund'schen Adjuvans intramuskulär injiziert. 20 Tage nach der ersten Injektion werden in 3 Tagesintervallen 4 i.v.-Injektionen mit 50 µg, 100 µg, 150 µg und 250 µg der Proteinpräparationen in 0,5 ml, ohne Adjuvans, injiziert. Eine Woche nach der letzten Immunisierung wurde Blut aus der Ohrvene entnommen und bei 4°C stehen gelassen. Das Serum wurde durch Zentrifugation bei 4000 x g, 15 min, gewonnen und in 0,5 ml Portionen bei -70°C eingefroren.

Immundiffusion:

Immundiffusionsuntersuchungen wurden mit Ausnahme der nachstehend beschriebenen Modifikation entsprechend der Methode nach Ouchterlony durchgeführt (O. Ouchterlony, Acta Pathol. Microbiol. Scand. (1949) 26: 507-515).

Die Immundiffusion wurde auf Glasplatten ausgeführt, die mit 1 % Agarose beschichtet wurden, die 1 % Triton X-100 in phosphatgepufferter Kochsalzlösung enthielt.

Die Vertiefungen für den Antikörper enthielten üblicherweise 20 $\mu$l Serum, die Antigenvertiefungen 1 - 5 $\mu$l der entsprechenden Proben. Die hochgereinigten Flagella-Antigen-Präparationen wurden in 0,1 % Natriumdodecylsulfat (SDS) desintegriert.

Intakte Flagellen können nur schwer in die Agarose eindrineen und durch die Behardlung mit dem Detergens wurde sichergestellt, daß die Antikörper mit den monomeren Antigenen reagieren. Triton X-100 verhindert in den Immundiffusionsplatten das Präzipitieren des Antiserums durch SDS. Immundiffusionsplatten werden in der Feuchtekammer bei 30 ° C 24 Stunden inkubiert.

Immunelektrophorese:

Die Immunelektrophorese wurde entsprechend den Vorschriften von B. Weeke durchgeführt (A Manual of Quantitative Immunoelectrophoresis, Methods and Application, Axelsen, Kroll, Weeke, eds., Universitetsforlaget, Oslo, 1973, p. 15 - 37).

Im Prinzip besteht dieses Verfahren darin, zunächst die elektrophoretische Trennung eines Proteingemisches (im vorstehenden Fall hochgereinigtes Flagella-Antigen) in einem gepufferten Agarosegel durch SDS-Gelelektrophorese vorzunehmen und nach dem Trennvorgang ein präzipitierendes Immunserum (im vorstehenden Fall Kaninchenantiserum gegen rohes und hochgereinigtes Flagella(H)-Antigen) parallel zur Wanderungsrichtung der getrennten Proteine in einem Trog einzubringen.

Antigen und Antiserum diffundieren durch das Agarosegel, das 1 % Triton X-100 enthielt, anschließend gegeneinander und an ihren Berührungsstellen entstehen bogenförmige Präzipitationslinien, deren Anzahl, Lage und Form einen Einklick in die Zusammensetzung des Antigengemisches geben.

Ergebnisse der Immundiffusion:

Die hochgereinigten Flagelline zeigten im Ouchterlony-Test jeweils eine einzige Präzipitatbande gegen ihr homologes Antiserum, gleichgültig, ob dieses Antiserum gegen eine rohe Flagella-Antigen-Präparation oder gegen das hochreine Flagellin gerichtet war.

Ergebnisse der Immunelektrophorese:

Immunelektrophorese mit den hochreinen Flagellinen der Pseudomonas aeruginosa-Stämme M-2, 1210, 5939, 5940, 5142, 170018 zeigte unter Verwendung homologer Antiseren, gegen das entsprechende reine Flagella-Antigen als auch gegen eine rohe Flagella-Antigen-Präparation gerichtet, eine einzige Präzipitatbande.

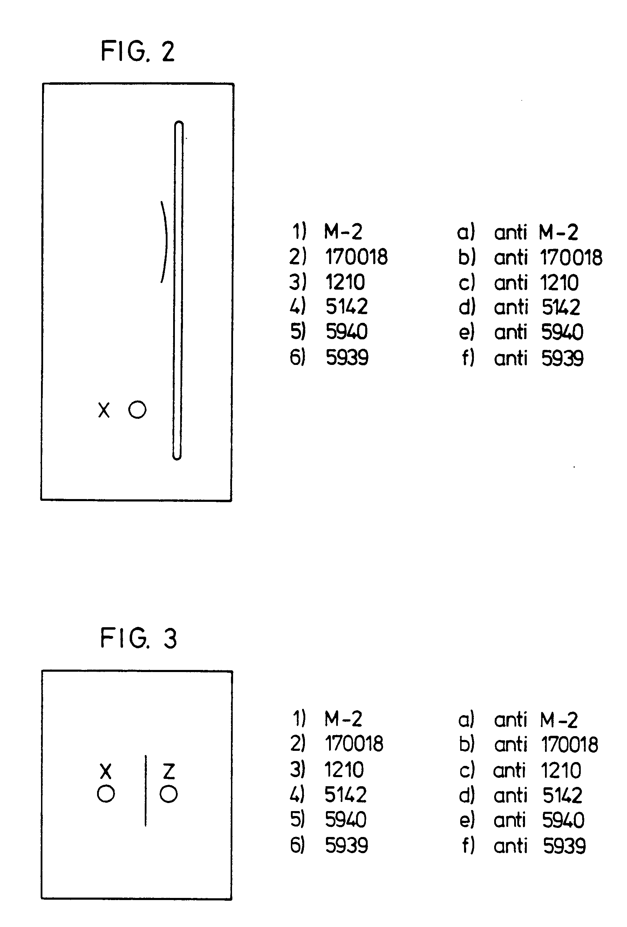

In Fig. 2 sind die Präzipitatbanden der eindimensionalen Immunelektrophorese nach Weeke und in Fig. 3 die Präzipitatbanden der Immundiffusion nach Ouchterlony dargestellt.

Wie aus Fig. 2 zu ersehen ist, wurden die Flagellinproben 1 entsprechend dem Stamm M-2, 2 entsprechend dem Stamm 170018, 3 entsprechend dem Stamm 1210, 4 entsprechend dem Stamm 5142, 5 entsprechend dem Stamm 5940 und 6 entsprechend dem Stamm 5939 jeweils in das Loch X des Gelstreifens aufgetragen und nach der Elektrophorese die entsprechenden Antisera (a - f) in den Schlitz pipettiert. Es entstand in allen Fällen, wie dargestellt, eine einzige Präzipitationsbande, was die Reinheit aller sechs Flagellinproben beweist.

Gemäß der Darstellung in Fig. 3 wurde für jeden Flagellentyp M-2, 170018, 1210, 5142, 5940 und 5939 je ein Test mit dem zugehörigen Antiserum durchgeführt. Die Flagellinprobe wurde jeweils im Loch X der Agarplatte aufgetragen und das entsprechende Antiserum in das Loch Z. Auch hier trat jeweils nur eine einzige starke Präzipitationsbande auf, was die Reinheit aller sechs Flagellinproben beweist.

**Patentansprüche**

1. Polydisperse native Pseudomonas-Flagella(H)-Antigene in polymerer Form mit
   (i) einem Diffusionskoeffizienten von D = 8,5 x $10^{-9}$ ($cm^2$/s),
   (ii) einem hydrodynamischen Radius von R = 2,5 x $10^{-5}$ cm,
   (iii) einer Dichte von d = 1,28 g/$cm^3$ und
   (iv) einer weitgehenden Freiheit von LPS, das ist ein Anteil von weniger als 1 %,
   welche Antigene aus monomeren Bestandteilen bestehen, wobei jeder monomere Bestandteil
   a) folgende Aminosäuren: Asparaginsäure (Asp), Threonin (Thr), Serin (Ser), Glutaminsäure (Glu), Glycin (Gly), Alanin (Ala), Valin (Val), Isoleucin (Ile), Leucin (Leu), Tyrosin (Tyr), Phenylalanin (Phe), Lysin (Lys), Arginin (Arg) und gegebenenfalls Tryptophan (Trp) enthält,
b) die N-terminale Aminosäuresequenz Alanin (Ala) - Leucin (Leu) - Threonin (Thr) - Valin (Val) - Asparagin (Asn) - Threonin (Thr) - Asparagin (Asn) - Isoleucin (Ile) - Alanin (Ala) - aufweist, und
c) ein Molekulargewicht zwischen 43.500 und 53.050 aufweisen und
d) frei sind von Prolin, Methionin, Halb-Cystin und Histidin.

2. Flagella(H)-Antigen vom H-Serotyp $a_0$, $a_3$, $a_4$ nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 64 : 33 : 35 : 42 : 44 : 68 : 29 : 29 : 37 : 3 : 10 : 19 : 15 enthält und ein Molekulargewicht von 43.500 aufweist.

3. Flagella(H)-Antigen vom K-Serotyp $a_0$, $a_3$ nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 69 : 35 : 38 : 44 : 47 : 73 : 30 : 30 : 60 : 3 : 12 : 21 : 16 enthält und ein Molekulargewicht von 46.700 aufweist.

4. Flagella(H)-Antigen vom H-Serotyp $a_0$ nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 50 : 49 : 49 : 49 : 89 : 37 : 29 : 44 : 5 : 14 : 17 : 16 enthält und ein Molekulargewicht von 52.720 aufweist.

5. Flagella(H)-Antigen vom H-Serotyp b nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 48 : 48 : 49 : 51 : 91 : 38 : 30 : 43 : 4 : 13 : 18 : 18 enthält und ein Molekulargewicht von 53.050 aufweist.

6. Flagella(H)-Antigen vom H-Serotyp $a_0$, $a_1$, $a_2$ nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 76 : 44 : 40 : 52 : 50 : 81 : 32 : 32 : 41 : 4 : 12 : 20 : 18 enthält und ein Molekulargewicht von 51.250 aufweist.

7. Flagella(H)-Antigen vom H-Serotyp $a_0$, $a_2$ nach Anspruch 1, dadurch gekennzeichnet, daß die monomere Form die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 68 : 41 : 37 : 46 : 44 : 73 : 29 : 29 : 37 : 3 : 10 : 16 : 16 enthält und ein Molekulargewicht von 45.900 aufweist.

8. Flagella(H)-Antigene nach einem der Ansprüche 1 bis 7, dadurch gekennzeichnet, daß sie gemäß dem Immunisierungsschema nach Lagenaur und Agabian in Kaninchen polyvalente oder monovalente Antikörper induzieren.

9. Flagella(H)-Antigene nach einem der Ansprüche 1 bis 8, dadurch gekennzeichnet, daß sie gegenüber polyvalenten und/oder monovalenten Antikörpern im ImmundiffusionsTest nach Ouchterlony oder im Immunelektrophorese-Test nach Weeke eine einzige Präzipitatbande bilden.

10. Verfahren zur Gewinnung von polydispersen nativen Pseudomonas aeruginosa Flagella(H)-Antigenen nach einem der Ansprüche 1 bis 7 aus Pseudomonas aeruginosa Bakterienkulturen, dadurch gekennzeichnet, daß die Bakterienkultur desintegriert, vorzugsweise einem "Shearing"-Prozeß unterworfen und fraktioniert wird, nach Zusatz eines Detergens einer chromatographischen Behandlung bzw. Reinigung unterworfen wird, wobei für die chromatographische Behandlung insbesondere ein Molekularsieb eingesetzt wird, welches mit dem Detergens äquilibriert ist, und daß das chromatographisch behandelte und gereinigte flagellare Antigen einer weiteren chromatographischen Reinigung zur Entfernung von vorhandenem Detergens unterworfen wird, wobei Flagella-Antigene mit einer Reinheit von mehr als 98 % und einem Anteil an pyrogenen Substanzen (LPS) von weniger als 1 % erhalten werden.

11. Verfahren nach Anspruch 10, dadurch gekennzeichnet, daß die Bakterienkultur vor der chromatographischen Behandlung einer Vorreinigung durch Abtrennen von Bakterien und mikroskopisch sichtbaren Bakterienteilchen unterworfen wird.

**12.** Verfahren nach Anspruch 11, dadurch gekennzeichnet, daß die Vorreinigung durch Zentrifugieren mit einer Zentrifugalbeschleunigung bis zu 5.000 x g vorgenommen wird, wobei das abgeschiedene Sediment verworfen wird.

**13.** Verfahren nach Anspruch 10, dadurch gekennzeichnet, daß als Detergens ein Salz der Gallensäure, insbesondere Deoxycholat, verwendet wird.

**14.** Verfahren nach Anspruch 10, dadurch gekennzeichnet, daß auch die zweite chromatographische Reinigung mit einem Molekularsieb, wie Sephadex™, oder einem Adsorptionsgel, wie BIO-BEADS SM 4™, durchgeführt wird.

**Claims**

**1.** Polydisperse native Pseudomonas flagellar (H) antigens in polymeric form having
  (i) a diffusion coefficient of $D = 8.5 \times 10^{-9}$ ($cm^2$/sec),
  (ii) a hydrodynamic radius of $R = 2.5 \times 10^{-5}$ cm,
  (iii) a density of $d = 1.28$ $g/cm^3$, and
  (iv) substantial freedom from LPS, i.e. a portion of less than 1%,
which antigens are comprised of monomeric components, each monomeric component
  a) containing the following amino acids: aspartic acid (Asp.), threonine (Thr), serine (Ser), glutamic acid (Glu), glycine (Gly), alanine (Ala), valine (Val), isoleucine (Ile), leucine (Leu), tyrosine (Tyr), phenyl alanine (Phe), lysine (Lys), arginine (Arg), and possibly tryptophane (Trp),
  b) comprising the N-terminal amino acid sequence alanine (Ala) - leucine (Leu) - threonine (Thr) - valine (Val) - asparagine (Asn) - threonine (Thr) - asparagine (Asn) - isoleucine (Ile) - alanine (Ala), and
  c) having a molecular weight of between 43,500 and 53,050, and
  d) being free from proline, methionine, semi-cystine and histidine.

**2.** Flagellar (H) antigen of the H-serotype $a_0$, $a_3$, $a_4$ according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 64 : 33 : 35 : 42 : 44 : 68 : 29 : 29 : 37 : 3 : 10 : 19 : 15 and has a molecular weight of 43,500.

**3.** Flagellar (H) antigen of the H-serotype $a_0$, $a_3$ according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 69 : 35 : 38 : 44 : 47 : 73 : 30 : 30 : 60 : 3 : 12 : 21 : 16 and has a molecular weight of 46,700.

**4.** Flagellar (H) antigen of the H-serotype $a_0$ according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 74 : 50 : 49 : 49 : 49 : 89 : 37 : 29 : 44 : 5 : 14 : 17 : 16 and has a molecular weight of 52,720.

**5.** Flagellar (H) antigen of the H-serotype b according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 74 : 48 : 48 : 49 : 51 : 91 : 38 : 30 : 43 : 4 : 13 : 18 : 18 and has a molecular weight of 53,050.

**6.** Flagellar (H) antigen of the H-serotype $a_0$, $a_1$, $a_2$ according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 76 : 44 : 40 : 52 : 50 : 81 : 32 : 32 : 41 : 4 : 12 : 20 : 18 and has a molecular weight of 51,250.

**7.** Flagellar (H) antigen of the H-serotype $a_0$, $a_2$ according to claim 1, characterised in that the monomeric form comprises the amino acids: aspartic acid, threonine, serine, glutamic acid, glycine, alanine, valine, isoleucine, leucine, tyrosine, phenyl alanine, lysine and arginine at a ratio of 68 : 41 : 37 : 46 : 44 : 73 : 29 : 29 : 37 : 3 : 10 : 16 : 16 and has a molecular weight of 45,900.

11

**8.** Flagellar (H) antigens according to one of claims 1 to 7, characterised in that they induce polyvalent or monovalent antibodies in rabbits according to the immunisation pattern according to Lagenaur and Agabian.

**9.** Flagellar (H) antigens according to one of claims 1 to 8, characterised in that they form a single precipitate band relative to polyvalent and/or monovalent antibodies in the immunodiffusion test according to Ouchterlony or in the immunoelectrophoresis test according to Weeke.

**10.** Method of recovering polydisperse native Pseudomonas aeruginosa flagellar (H) antigens according to one of claims 1 to 7 from Pseudomonas aeruginosa bacterial cultures, characterised in that the bacterial culture is disintegrated, preferably subjected to a shearing procedure and fractionated, upon the addition of a detergent is subjected to a chromatographic treatment or purification, respectively, wherein, for the chromatographic treatment in particular a molecular sieve is used which has been equilibrated with the detergent, and that the chromatographically treated and purified flagellar antigen is subjected to a further chromatographic purification so as to remove detergent present, wherein the flagellar antigens are obtained in a purity of more than 98% and with a portion of pyrogenic substances (LPS) of less than 1%.

**11.** Method according to claim 10, characterised in that the bacterial culture is subjected to a pre-purification by separating bacteria and miscroscopically visible bacteria particles prior to subjecting it to the chromatographic treatment.

**12.** Method according to claim 11, characterised in that the pre-purification is effected by centrifuging with a centrifugal acceleration of up to 5,000 x g, the separated sediment being discarded.

**13.** Method according to claim 10, characterised in that as the detergent a salt of the bile acid, in particular deoxycholate, is used.

**14.** Method according to claim 10, characterised in that also the second chromatographic purification is carried out with a molecular sieve, such as Sephadex™, or an adsorption gel, such as BIO-BEADS SM 4™.

**Revendications**

**1.** Antigènes H de flagelles de Pseudomonas natifs polydispersés sous une forme polymère ayant :
(i) un coefficient de diffusion D = 8,5 x $10^{-9}$ (cm$^2$/s),
(ii) un rayon hydrodynamique R = 2,5 x $10^{-5}$ cm,
(iii) une densité d = 1,28 g/cm$^3$ et
(iv) une absence quasi totale de LPS, c'est-à-dire une proportion inférieure à 1 %,
lesquels antigènes sont constitués par des composants monomères, chaque composant monomère
a) contenant tes acides aminés suivants : acide asparagique (Asp), thréonine (Thr), sérine (Ser), acide glutamique (Glu), glycine (Gly), alanine (Ala), valine (Val), isoleucine (Ile), leucine (Leu), tyrosine (Tyr), phénylalanine (Phe), lysine (Lys), arginine (Arg) et, le cas échéant, tryptophane (Trp),
b) présentant la séquence d'acides aminés N terminale alanine (Ala)-leucine (Leu)-thréonine (Thr)-valine (Val)-asparagine (Asn)-thréonine (Thr)-asparagine (Asn)-isoleucine (Ile)-alanine (Ala), et
c) présentent un poids moléculaire compris entre 43 500 et 53 050 et
d) sont exempts de proline, de méthionine, d'hémi-cystine et d'histidine.

**2.** Antigène H de flagelles du sérotype H a$_0$, a$_3$, a$_4$ selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 64 : 33 : 35 : 42 : 44 : 68 : 29 : 29 : 37 : 3 : 10 : 19 : 15 et présente un poids moléculaire de 43 500.

**3.** Antigène H de flagelles du sérotype H a$_0$, a$_3$ selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 69 : 35 : 38 : 44 : 47 : 73 : 30 : 30 : 60 : 3 : 12 : 21 : 16 et présente un poids moléculaire de 46 700.

12

**4.** Antigène H de flagelles du sérotype H $a_0$ selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 74 : 50 : 49 : 49 : 49 : 89 : 37 : 29 : 44 : 5 : 14 : 17 : 16 et présente un poids moléculaire de 52 720.

**5.** Antigène H de flagelles du sérotype H b selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 74 : 48 : 48 : 49 : 51 : 91 : 38 : 30 : 43 : 4 : 13 : 18 : 18 et présente un poids moléculaire de 53 050.

**6.** Antigène H de flagelles du sérotype H $a_0$, $a_1$, $a_2$ selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 76 : 44 : 40 : 52 : 50 : 81 : 32 : 32 : 41 : 4 : 12 : 20 : 18 et présente un poids moléculaire de 51 250.

**7.** Antigène H de flagelles du sérotype H $a_0$, $a_2$ selon la revendication 1, caractérisé en ce que la forme monomère contient les acides aminés : acide asparagique, thréonine, sérine, acide glutamique, glycine, alanine, valine, isoleucine, leucine, tyrosine, phénylalanine, lysine et arginine dans le rapport de 68 : 41 : 37 : 46 : 44 : 73 : 29 : 29 : 37 : 3 : 10 : 16 : 16 et présente un poids moléculaire de 45 900.

**8.** Antigènes H de flagelles selon une des revendications 1 à 7, caractérisés en ce qu'ils induisent chez le lapin des anticorps monovalents ou polyvalents selon le schéma d'immunisation de Lagenaur et Agabian.

**9.** Antigènes H de flagelles selon l'une des revendications 1 à 8, caractérisés en ce qu'ils forment une seule bande de précipité vis-à-vis des anticorps polyvalents et/ou monovalents dans le test d'immunodiffusion selon Ouchterlony ou dans le test d'immunoélectrophorèse selon Weeke.

**10.** Procédé d'obtention d'antigènes H de flagelles de Pseudomonas aeruginosa natifs polydispersés selon l'une des revendications 1 à 7, à partir de cultures bactériennes de Pseudomonas aeruginosa, caractérisé en ce que la culture bactérienne est désintégrée, soumise de préférence à un procédé de "shearing" et fractionnée, soumise après l'addition d'un détergent à un traitement ou une purification chromatographiques, le traitement chromatographique utilisant notamment un tamis moléculaire équilibré par le détergent, et en ce que l'antigène flagellaire traité et purifié par chromatographie est soumis à une seconde purification chromatographique ayant pour but de retirer le détergent présent, les antigènes de flagelles ainsi obtenus ayant une pureté supérieure à 98 % et une proportion de substances pyrogènes (LPS) inférieure à 1 %.

**11.** Procédé selon la revendication 10, caractérisé en ce que la culture bactérienne est soumise avant le traitement chromatographique à une purification préalable par séparation de bactéries et de particules bactériennes visibles au microscope.

**12.** Procédé selon la revendication 11, caractérisé en ce que la purification préalable est opérée par centrifugation pour une accélération centrifuge maximale de 5 000 x g, le sédiment séparé étant rejeté.

**13.** Procédé selon la revendication 10, caractérisé en ce qu'on utilise comme détergent un sel de l'acide biliaire, notamment un désoxycholate.

**14.** Procédé selon la revendication 10, caractérisé en ce que la seconde purification chromatographique est opérée également à l'aide d'un tamis moléculaire tel que Sephadex ™ ou d'un gel d'adsorption tel que 6I0-BEADS SM 4 ™.

FIG. 1

## FIG. 2

| 1) M-2 | a) anti M-2 |
|---|---|
| 2) 170018 | b) anti 170018 |
| 3) 1210 | c) anti 1210 |
| 4) 5142 | d) anti 5142 |
| 5) 5940 | e) anti 5940 |
| 6) 5939 | f) anti 5939 |

## FIG. 3

| 1) M-2 | a) anti M-2 |
|---|---|
| 2) 170018 | b) anti 170018 |
| 3) 1210 | c) anti 1210 |
| 4) 5142 | d) anti 5142 |
| 5) 5940 | e) anti 5940 |
| 6) 5939 | f) anti 5939 |